# EUROPEAN PATENT APPLICATION

(11) **EP 4 807 757 A1**
(43) Date of publication of application: **16.09.2026**
(21) Application number: 25225957.7
(22) Date of filing: 19.12.2025
(51) Int. Cl.: G16H 20/70, G16H 80/00, A61B 5/16

(54) **METHOD AND SYSTEM FOR REAL-TIME EMOTIONAL STATE SHIFT DETECTION AND ADAPTIVE RESPONSE GENERATION**

(30) Priority: 11.03.2025 IN 202521021914
(71) Applicant: Tata Consultancy Services Limited, Maharashtra (IN)
(72) Inventor: RAO, Shilpa Yadukumar, 603103 Chennai, Tamil Nadu (IN); SENTHIL KUMAR, Sanju, 603103 Chennai, Tamil Nadu (IN); PAATIL, Suchitaa, 411057 Pune, Maharashtra (IN); ANAND, Taruna, 110001 New Delhi, Delhi (IN)
(74) Representative: Goddar, Heinz J.

(57) **Abstract**

Current emotional detection technologies often struggle to consistently identify emotional state shifts in real-time, particularly in noisy environments where signals fluctuate due to minor external changes, and also fail to provide a robust mechanism that can account for and filter out these transient signals. Method and system disclosed herein perform real-time emotional state shift detection of a user and adaptive response generation. The system captures and processes a multi-modal input data in real time, during which weight of each multi-modal input data is determined based on a sensitivity factor that is determined by performing dynamic sensitivity adjustment for each multi-modal input data. An adaptive question is generated for the facilitator and the response is collected. The user is made to play a generative game until the user is identified to be in a pre-defined state. Further, a customized interview structure is generated for the user.

## Description

### CROSS-REFERENCE TO RELATED APPLICATIONS AND PRIORITY

The present application claims priority from Indian application no. 202521021914, filed on March 11, 2025.

### TECHNICAL FIELD

The disclosure herein generally relates to user emotional state detection technologies, and, more particularly, to a real-time emotional state shift detection of a user and adaptive response generation.

### BACKGROUND

Emotional detection can be used in a variety of applications. For example, in medical examination, educational purposes, change management, wellbeing and so on, detected user emotional states can be useful. Current emotional detection technologies often struggle to consistently identify emotional states in real-time, particularly in noisy environments where signals fluctuate due to minor external changes, and also fail to provide a robust mechanism that can account for and filter out these transient signals. Many emotion detection systems rely on isolated data streams (e.g., only heart rate or facial expressions) without validating results across multiple modalities. Cross-validation across different physiological and behavioral signals is often overlooked or handled separately, resulting in inconsistent state identification. Many systems misinterpret transient reactions as meaningful emotional states, resulting in frequent misclassification and inaccurate adjustments. There is currently no effective mechanism that distinguishes transient emotional responses from stable, sustained states.

### SUMMARY

Embodiments of the present disclosure present technological improvements as solutions to one or more of the above-mentioned technical problems recognized by the inventors in conventional systems. For example, in one embodiment, a processor implemented method is provided. The method includes: capturing, via one or more hardware processors, a multi-modal input data in real time, at each time stamp over a period of time, from a user and a facilitator during an interactive session using a set of sensors; pre-processing, via the one or more hardware processors, the multi-modal input data to generate a pre-processed input data, wherein the pre-processing comprises of filtering noise in the multi-modal input data using a set of filtering techniques; computing, via the one or more hardware processors, a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics; generating, via the one or more hardware processors, an adaptive question for the facilitator based on a cumulative alignment score indicating rapport between the user and the facilitator, a persistent emotional state detected based on the computed weight of each multi-modal input data, and a predicted future emotional state; displaying, via the one or more hardware processors, a generative game for the user to play based on the detected persistent emotional state, wherein the user plays the generative game until the user is identified to be in a pre-defined state; and generating, via the one or more hardware processors, a customized interview structure for the user, based on the adaptive question generated prior to displaying the generative game, the detected persistent state, a user response for the displayed adaptive game, one or more adaptive questions generated based on the user response for the displayed adaptive game, and the anchor for one or more states identified to be shifted.

In an aspect of the method, the multi-modal input data comprises micro-expressions, gestures, heart rate, breather rate, breathe volume, eye ball tracking and eye gesture data, and one or more additional parameters representing emotional state of the user.

In another aspect of the method, the sensitivity factor is represented as: ${S}_{i} \left(t\right) = \left\{\left({α}_{i}∗\left‖{X}_{i}\left(t\right)-{B}_{i}\left(t\right)\right‖\right)/{σ}_{i}{\left(t\right)}^{2}+∈\right\} + \left\{{β}_{i}/\left(\begin{matrix}1 + \\ Noiselevel \left({X}_{i}\left(t\right)\right)\end{matrix}\right)\right\} ,$ where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data i over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$

In another aspect of the method, the persistent emotional state for a pre-defined time interval is detected by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising: calculating a topological persistence score of the emotional state at each time instant by mapping the emotional states to a topological space, wherein the emotional state and a congruence score at a time instant is determined based on the computed weight for each multi-modal input data using a unified state model, wherein the congruence score is represented as: $CS \left(E\right) = 1 / N {\sum }_{i = 1}^{n} \left(1-\left\{\left|{X}_{i}-{B}_{i}\right|\left./σi\right)\right\}\right) .$ *Wᵢ*, where *Xᵢ* is the current value of
the modality *i, Bᵢ* is the baseline of the modality *i, σi* is the standard deviation of *i*, and *Wᵢ* is the weight assigned to modality *i* based on its reliability.

In another aspect of the method, the cumulative alignment score is generated from the weight computed for the multi-modal input data of the user and the facilitator using a dynamic time warping and phase alignment technique.

In another aspect of the method, the future emotional state is predicted at each time instant from the weight computed for the multi-modal input data of the user, the adaptive question generated at the previous time instant, a user response generated at the previous time instant, and an anchor generated at the previous instant.

In another aspect, a system is provided. The system includes one or more hardware processors, a communication interface, and a memory storing a plurality of instructions. The plurality of instructions cause the one or more hardware processors to: capture a multi-modal input data in real time, at each time stamp over a period of time, from a user and a facilitator during an interactive session using a set of sensors; pre-process the multi-modal input data to generate a pre-processed input data, wherein the pre-processing comprises of filtering noise in the multi-modal input data using a set of filtering techniques; compute a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics; generate an adaptive question for the facilitator based on a cumulative alignment score indicating rapport between the user and the facilitator, a persistent emotional state detected based on the computed weight of each multi-modal input data, and a predicted future emotional state; display a generative game for the user to play based on the detected persistent emotional state, wherein the user plays the generative game until the user is identified to be in a pre-defined state; and generate a customized interview structure for the user, based on the adaptive question generated prior to displaying the generative game, the detected persistent state, a user response for the displayed adaptive game, one or more adaptive questions generated based on the user response for the displayed adaptive game, and the anchor for one or more states identified to be shifted.

In an aspect of the system, the multi-modal input data comprises micro-expressions, gestures, heart rate, breather rate, breathe volume, eye ball tracking and eye gesture data, and one or more additional parameters representing emotional state of the user.

In another aspect of the system, the sensitivity factor is represented as: ${S}_{i} \left(t\right) = \left\{\left({α}_{i}∗\left‖{X}_{i}\left(t\right)-{B}_{i}\left(t\right)\right‖\right)/{σ}_{i}{\left(t\right)}^{2}+∈\right\} + \left\{{β}_{i}/\left(\begin{matrix}1 + \\ Noiselevel \left({X}_{i}\left(t\right)\right)\end{matrix}\right)\right\} ,$ where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data *i* over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\}$*.*

In another aspect of the system, the one or more hardware processors are configured to detect the persistent emotional state for a pre-defined time interval by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising: calculating a topological persistence score of the emotional state at each time instant by mapping the emotional states to a topological space, wherein the emotional state and a congruence score at a time instant is determined based on the computed weight for each multi-modal input data using a unified state model, wherein the congruence score is represented as: CS(E) = $1 / N {\sum }_{i = 1}^{n} \left(1-\left\{\left|{X}_{i}-{B}_{i}\right|\left./σi\right)\right\}\right)$*. Wᵢ*, where *Xᵢ* is the current value of the modality *i, Bᵢ* is the baseline of the modality *i, σi* is the standard deviation of *i,* and *Wᵢ* is the weight assigned to modality *i* based on its reliability.

In another aspect of the system, the one or more hardware processors are configured to generate the cumulative alignment score from the weight computed for the multi-modal input data of the user and the facilitator using a dynamic time warping and phase alignment technique.

In another aspect of the system, the one or more hardware processors are configured to predict the future emotional state at each time instant from the weight computed for the multi-modal input data of the user, the adaptive question generated at the previous time instant, a user response generated at the previous time instant, and an anchor generated at the previous instant.

In yet another aspect, there are provided one or more non-transitory machine-readable information storage mediums comprising one or more instructions which when executed by one or more hardware processors cause: capturing a multi-modal input data in real time, at each time stamp over a period of time, from a user and a facilitator during an interactive session using a set of sensors; pre-processing the multi-modal input data to generate a pre-processed input data, wherein the pre-processing comprises of filtering noise in the multi-modal input data using a set of filtering techniques; computing a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics; generating an adaptive question for the facilitator based on a cumulative alignment score indicating rapport between the user and the facilitator, a persistent emotional state detected based on the computed weight of each multi-modal input data, and a predicted future emotional state; displaying a generative game for the user to play based on the detected persistent emotional state, wherein the user plays the generative game until the user is identified to be in a pre-defined state; and generating a customized interview structure for the user, based on the adaptive question generated prior to displaying the generative game, the detected persistent state, a user response for the displayed adaptive game, one or more adaptive questions generated based on the user response for the displayed adaptive game, and the anchor for one or more states identified to be shifted.

In an aspect of the one or more non-transitory computer readable medium, the multi-modal input data comprises micro-expressions, gestures, heart rate, breather rate, breathe volume, eye ball tracking and eye gesture data, and one or more additional parameters representing emotional state of the user.

In another aspect of the one or more non-transitory computer readable medium, the sensitivity factor is represented as: 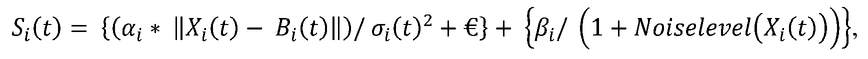 where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data i over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$

In another aspect of the one or more non-transitory computer readable medium, the one or more hardware processors are configured to detect the persistent emotional state for a pre-defined time interval by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising: calculating a topological persistence score of the emotional state at each time instant by mapping the emotional states to a topological space, wherein the emotional state and a congruence score at a time instant is determined based on the computed weight for each multi-modal input data using a unified state model, wherein the congruence score is represented as: $CS \left(E\right) = 1 / N {\sum }_{i = 1}^{n} \left(1-\left\{\left|{X}_{i}-{B}_{i}\right|\left./σi\right)\right\}\right)$. *Wᵢ*, where *Xᵢ* is the current value of the modality *i, Bᵢ* is the baseline of the modality *i, σi* is the standard deviation of i, and *Wᵢ* is the weight assigned to modality i based on its reliability.

In another aspect of the one or more non-transitory computer readable medium, the one or more hardware processors are configured to generate the cumulative alignment score from the weight computed for the multi-modal input data of the user and the facilitator using a dynamic time warping and phase alignment technique.

In another aspect of the one or more non-transitory computer readable medium, the one or more hardware processors are configured to predict the future emotional state at each time instant from the weight computed for the multi-modal input data of the user, the adaptive question generated at the previous time instant, a user response generated at the previous time instant, and an anchor generated at the previous instant.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for emotional state shift detection of a user, according to some embodiments of the present disclosure.
FIG. 2 is a flow diagram depicting steps involved in the process of for emotional state shift detection of a user, by using the system of FIG. 1, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Emotional detection can be used in a variety of applications. For example, in medical examination, educational purposes and so on, detected user emotional states can be useful. Current emotional detection technologies often struggle to consistently identify emotional states in real-time, particularly in noisy environments where signals fluctuate due to minor external changes, and also fail to provide a robust mechanism that can account for and filter out these transient signals. Many emotion detection systems rely on isolated data streams (e.g., only heart rate or facial expressions) without validating results across multiple modalities. Cross-validation across different physiological and behavioral signals is often overlooked or handled separately, resulting in inconsistent state identification. Many systems misinterpret transient reactions as meaningful emotional states, resulting in frequent misclassification and inaccurate adjustments. There is currently no effective mechanism that distinguishes transient emotional responses from stable, sustained states.

In order to address these challenges a method and system for emotional state detection using real-time monitoring of a user is provided. In this method, a multi-modal input data is captured in real time, at each time stamp over a period of time, from a user and a facilitator during an interactive session using a set of sensors. Further, the multi-modal input data is pre-processed to generate a pre-processed input data, wherein the pre-processing comprises of filtering noise in the multi-modal input data using a set of filtering techniques. Further, a weight of each multi-modal input data of the user and the facilitator is computed based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics. Further, an adaptive question is generated for the facilitator based on a cumulative alignment score indicating rapport between the user and the facilitator, a persistent emotional state detected based on the computed weight of each multi-modal input data, and a predicted future emotional state. Further, a generative game for the user to play is displayed based on the detected persistent emotional state, wherein the user plays the generative game until the user is identified to be in a pre-defined state. Further, a customized interview structure for the user is generated based on the adaptive question, the detected persistent state, a user response for the displayed adaptive game, and the anchor for one or more states identified to be shifted.

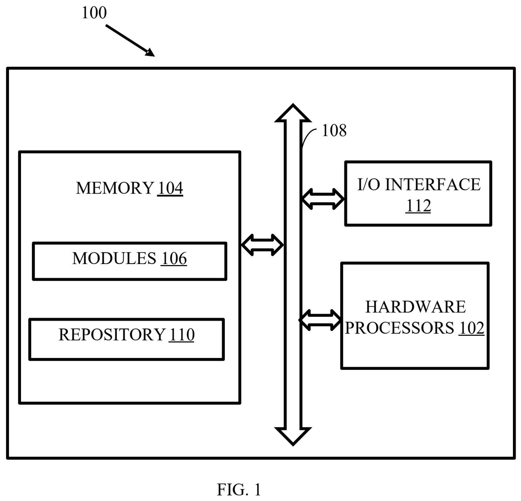

Referring now to the drawings, and more particularly to FIG. 1 through FIG. 2, where similar reference characters denote corresponding features consistently throughout the figures, there are shown preferred embodiments and these embodiments are described in the context of the following exemplary system and/or method.

FIG. 1 illustrates an exemplary system 100 for emotional state prediction of a user, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of the emotional state prediction of the user, being performed by the system of FIG. 1. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the emotional state prediction of the user.

The data repository (or repository) 110 may include a plurality of abstracted piece of code for refinement and data that is processed, received, or generated as a result of the execution of the plurality of modules in the module(s) 106.

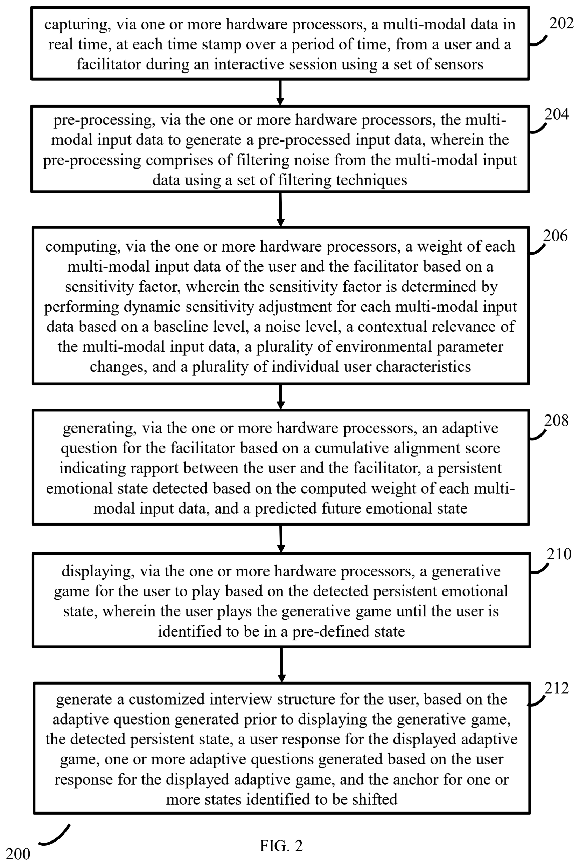

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the flow diagram in FIG. 2.

FIG. 2 is a flow diagram depicting steps involved in the process of for emotional state prediction of a user, by using the system 100 of FIG. 1, according to some embodiments of the present disclosure.

In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of method 200, the system 100 captures, via the one or more hardware processors 102, a multi-modal input data in real time at each time stamp over a period of time, from a user and a facilitator during an interactive session using a set of sensors. The user and the facilitator maybe engaged in any type of discussion, for an assessment purpose. For example, the user and the facilitator are part of an interview process, where the user is the person being interviewed, and the facilitator is the person interviewing the user. In another example, the facilitator maybe a qualified doctor and the user maybe a person whose mental state is being assessed by the doctor. In various embodiments, the interaction between the facilitator and the user is online, over any video calling application such as but not limited to MS Teams^{®}, Google Meet^{™}, Zoom ^{®}, and so on, or is a direct meeting. In any of these scenarios, the conversation/interaction between the user and the facilitator is being captured using video recording device, i.e., a camera, along with the corresponding voice, and is transmitted to the system 100 for further processing. In an embodiment, along with the audio/video, other data such as but not limited to heart rate of the user, eyeball movements, and breathe rate, of the user as well as of the facilitator also maybe captured using an appropriate sensor(s) and are processed further.

Further, at step 204 of the method 200, the system 100 pre-processes, via the one or more hardware processors 102, the multi-modal input data to generate a pre-processed input data. The pre-processing comprises of filtering noise from the multi-modal input data using a set of filtering techniques. Some examples of the filtering techniques used are, Kalman filtering, Wiener filtering, and spectral subtraction.

Further, at step 206 of the method 200, the system 100 dynamically computes weights to each of the different modalities of data that is collected, i.e., the audio, video, heart rate, eyeball movements, breathe rate and so on, based on factors such as but not limited to real-time data quality, environmental changes (e.g., noise, lighting), and individual user characteristics. The weights are dynamically assigned are adjusted based on a sensitivity factor, by performing dynamic sensitivity adjustment for each multi-modal input data based on a baseline level, a noise level, a contextual relevance of the multi-modal input data, a plurality of environmental parameter changes, and a plurality of individual user characteristics. For example, in a browser-based session, the system 100 is detecting the emotional state of a user engaged in a coaching session. If the user shifts from a calm to a stressed state (detected via increased heart rate and change in voice tone), the sensitivity factor for heart rate and voice tonality is increased to capture this transition accurately. If a background noise suddenly increases, the system 100 detects this noise and reduces the sensitivity of voice inputs temporarily, relying more on micro-expressions and heart rate until the noise subsides. The sensitivity factor is computed as: 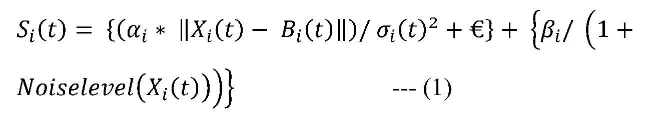 where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data i over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$

The weights are updated in real-time based on changes in the sensitivity factor, ensuring that the influence of each modality is proportional to its reliability and relevance at any given moment.

The system 100 also performs contextual recalibration of one or more of the parameters used in equation (1) when specific environmental changes or user behaviour shifts are detected. An example of the user behaviour shifts is the user moving from an environment that is marked as a quiet environment to an environment that is marked as a noisy environment. The recalibration involve one or more of: a) Temporarily reducing *αᵢ* and increasing β*ᵢ* for modalities that are highly sensitive to the detected context (e.g., voice inputs in a noisy environment)m where information on the sensitivity of various modalities with respect to different defined contexts are preconfigured and/or dynamically configured with the system 100, b) Re-initializing the baseline *Bᵢ*(*t*) if a consistent pattern of deviation is observed, indicating a potential change in user's emotional or physiological state.

In an embodiment, an adaptive baseline updation is performed by the system 100 to update value of the baseline *Bᵢ*(*t*), using a weighted moving average formula: ${B}_{i} \left(t+1\right) = \left(1-γ\right) ∗ {B}_{i} \left(t\right) + γ ∗ {X}_{i} \left(t\right)$ where, *γ* is a learning rate that controls how quickly the baseline adapts to new inputs. A high *γ* value (i.e., higher than a threshold of *γ*) means the baseline updates quickly in response to changes, whereas a low *γ* value (i.e., lower than the threshold of *γ*) means slower adaptation, ensuring stability. If the noise level *Noiselevel*(*Xᵢ*(*t*)) exceeds a predefined threshold, the sensitivity of the affected modality is automatically reduced to prevent erroneous emotional state classifications.

The system 100 receives predefined state shifts and emotional state classifications from a Micro expression Application Programming Interface (API) and a voice analysis module (not shown in FIGS.). Each state shift is tagged with a confidence score provided by one or more external modules (e.g., inputs confidence in detected emotional states). These inputs are treated as initial state indicators that need to be validated against additional physiological data (e.g., heart rate, breath rate). To determine the state shifts for the validation purpose, the system 100 relies on integration of various parameters. For example, Heart Rate and Breath Rate data are integrated to assess physiological congruence with the emotional states provided by Micro expression Analysis and voice analysis. A Pearson's Correlation Coefficient is applied to measure the linear relationship between heart rate variability (HRV), breath rate changes, and the emotional states detected. The correlation is computed as: $ρ \left(E\right) = \left(Cov\left({X}_{i}, Y\right)/{σX}_{i}.σY\right)$ where, *Cov* (*Xᵢ, Y*) is the covariance between modality *Xᵢ* (e.g., heart rate variability) and the external emotional state *Y,* and *σXᵢ.σY* are the standard deviations of the modality and the emotional state, respectively. A high *ρ*(*E*) indicates strong agreement between the physiological data and the detected emotional state.

A Congruence Score (CS) is calculated to determine the agreement between the external state shift and additional modalities, as: $CS \left(E\right) = 1 / N {\sum }_{i = 1}^{n} \left(1-\left\{\left|{X}_{i}-{B}_{i}\right|/σi\right\}\right) . {W}_{i}$ where,
*Xᵢ* is the current value of the modality *i.*
*Bᵢ* is the baseline of the modality *i.*
*σi* is the standard deviation of *i.*
*Wᵢ* is the weight assigned to modality *i,* based on its reliability.

The congruence score quantifies how closely the current physiological state matches an expected state for the detected emotion. High congruence indicates that the state shift is valid and stable. If the Congruence Score CS(E) is lower than a threshold of congruence score, it indicates a discrepancy between micro expression/voice analysis and physiological data, and then the system 100 dynamically adjusts the sensitivity of each modality, by: decreasing sensitivity of modalities that have been identified as less reliable (e.g., reduce reliance on breath rate if inconsistent), and increasing sensitivity modalities that have been identified reliable. The adjusted sensitivity factors Si(t) are then used for subsequent state classifications.

The system 100 may use also use a Bidirectional Long Short-Term Memory (Bi-LSTM) network to track sequential patterns and shifts in emotional states over time. The Bi-LSTM model uses previous and future context to refine state detection. The Bi-LSTM model is trained using state shift simulations generated using a Generative Adversarial Network (GAN). A generator of the GAN creates hypothetical state shifts, and a discriminator of the GAN validates these shifts against real-time data, ensuring that detected shifts are plausible. Further, a Q-Learning-based Reinforcement Learning (RL) model is used to optimize sensitivity adjustments. The RL model is needed to learn in longer term from the interaction between the system 100 and user to identify which interventions and questions linguistics patterns lead to desired emotional outcomes in the context over time. The RL model helps the system 100 to continuously adapt, improving its strategy based on the user's emotional shift and evolving conditions. An RL agent dynamically adjusts the sensitivity of each modality based on feedback from state validations, reducing reliance on less consistent inputs. The RL agent learns optimal sensitivity settings based on rewards received for accurate state validations: $Q \left({S}_{i}, a\right) < Q \left({S}_{i}, a\right) + α \left\{R\left(t\right)+γ maxQ\left({S}_{i}\left(t+1\right),a′\right)-Q\left({S}_{i}, a\right)\right\}$ where,
*Q*(*Sᵢ, a*) *: Action value function*
*α* is the learning rate
*γ* is the discount factor
R reward function after taking action *α* in state s
*Sᵢ*(*t +* 1) is next state
*max* 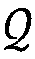 (*Sᵢ*(*t +* 1), *a*') is maximum predicted Q*_{w}* value for the next state. The RL model updates current Q-value estimate by taking into account a best possible future reward from next state.

Further, at step 208 of the method 200, the system 100 generates, via the one or more hardware processors 102, an adaptive question for the facilitator based on a cumulative alignment score indicating rapport between the user and the facilitator, a persistent emotional state detected based on the computed weight of each multi-modal input data, and a predicted future emotional state. The future emotional state is predicted at each time instant from the weight computed for the multi-modal input data of the user, the adaptive question generated at the previous time instant, a user response generated at the previous time instant, and an anchor generated at the previous instant. The system 100 maps the persistent emotional state onto a topological structure using persistent homology.

The rapport between the user and the facilitator is evaluated by the system 100 using the multimodal data inputs such as breath rate, posture, speed of words, and linguistic patterns. The system 100 dynamically adapts the facilitator's behaviour (e.g., adjusting breath rate) to match the participant's state and uses a micro-expression analysis to continuously update rapport scores. The system 100 visualizes this information for the facilitator, providing actionable feedback to improve engagement and alignment. The system 100 uses sophisticated algorithms to match, measure, and align the participant's and facilitator's behaviours based on these inputs, and provides actionable feedback to the facilitator to improve engagement and interaction quality. This dynamic alignment fosters better emotional connection and mutual understanding between the user and the facilitator.

For the rapport evaluation, the system 100 receives the following streams of data:
- Micro-Expression Feed: Facial micro-expressions are captured via a micro expression API, providing insights into transient emotional states (e.g., surprise, joy, discomfort) of the user and the facilitator.
- Breath Rate Analysis: Breath rate data is captured with video and audio and is made available to the system 100 via an API.
- Voice Speed Analysis: Voice speed (words per minute, pauses) is analyzed using a lightweight model and is made available to the system 100 via an API.

The system 100 then performs a Generative AI (Gen-AI) -Based Linguistic Analysis on the collected streams of data. The Gen-AI model processes spoken words to identify certain words defined and classified as "magic words" that hold emotional or cognitive significance for the user and the facilitator. The magic words are specific key phrases and emotionally significant words that are identified by the Generative AI and are stored and weighted based on their frequency and contextual relevance. These magic words are contextual words that may signal values, beliefs, or emotional triggers (e.g., "family," "success," "challenge"). The Gen-AI model also analyses sentence structures and phrasing patterns to identify shifts in emotional tone, hesitations, or linguistic markers of stress or excitement, and also identifies words that are frequently used. The system 100 then matches the user's multimodal inputs with corresponding inputs from the facilitator. This includes:
▪ Micro-Expression Matching: The intensity and duration of the user's micro-expressions are compared with those of the facilitator's to evaluate emotional mirroring.
▪ Breath Rate Synchronization: The breath rates of the user as well as of the evaluator are dynamically measured and aligned. The system 100 suggests adjusting the facilitator's breath rate to achieve synchronization if the user's breath rate is more erratic or calm.
▪ Voice Speed Alignment: The system 100 suggests adjusting the facilitator's voice speed to match the user's pacing (e.g., speaking more slowly if the participant is pausing frequently).
▪ Magic Word Usage: The system 100 tracks the use of magic words by both parties. If the participant frequently uses a specific word (e.g., "family"), the system 100 suggests incorporating this word into the facilitator's responses to create common ground and enhance rapport.

For the rapport building, the system 100 aligns each modality (micro-expressions, breath rate, voice speed) individually between the user and the facilitator using a Dynamic Time Warping (DTW) algorithm. The DTW provides optimal alignment paths between the sequences of both parties, ensuring accurate matching despite temporal differences.

The alignment score for modality i is defined as: ${Alignment}_{i} \left(t\right) = \left(1\K\right) {\sum }_{k = 1}^{k} \left\{1-\left.\left(\begin{matrix}\left(\left|Mi,user\left(tk\right)-Mi,facilitator\left(tk\right)\right|\right) / \\ max \left(Mi,user\left(tk\right),Mi facilitator\left(tk\right)\right) + ε\end{matrix}\right)\right)\right\}$ where,
∘ Mi, user(tk), Mi facilitator(tk) are the values of modality *i* (e.g., micro-expression intensity, breath rate) for the user and facilitator at time tk
∘ ε is a small constant to prevent division by zero.
∘ *K* is the total number of time steps in the alignment path.

A cumulative multimodal alignment score R(t) is computed by summing the alignment scores of each modality, weighted by their relative importance at time t: $R \left(t\right) = {\sum }_{i = 1}^{n} {W}_{i} \left(t\right) {Alignment}_{i} \left(t\right)$ where,
∘ *Wᵢ*(*t*) is the dynamic weight of modality *i* at time *t,* adjusted based on the consistency and reliability of each input.
∘ This cumulative score is visualized as a real-time rapport score, allowing the facilitator to see how well they are aligning with the user across all modalities.

Further, the system 100 performs semantic matching of the Magic Words. In this process, the system 100 tracks magic words identified by the generative AI model and compares their use between the user and the facilitator using a semantic similarity measure (e.g., cosine similarity): $MagicWordScore \left(t\right) = Cosθ = \left\{{\sum }_{k} TF - {IDF}_{user}^{k} . TF - {IDF}_{facilitator}^{k}\right\} / \left\{\left‖\begin{matrix}TF - \\ {IDF}_{user}\end{matrix}\right‖.\left‖TF-{IDF}_{facilitator}\right‖\right\}$ where, ${\sum }_{k} TF - {IDF}_{user}^{k} and TF - {IDF}_{facilitator}^{k}$
are the TF-IDF values of the word *k* for the user and facilitator, respectively.

If the semantic alignment score for magic words is lower than a threshold, the system 100 suggests incorporating specific words into the facilitator's responses. This suggestion is based on the frequency, context, and emotional weight of words used by the user. The suggested words may be displayed on a facilitator's screen as a dynamic feedback, guiding them to use these words in subsequent interactions to increase rapport.

The system 100 achieves breath rate synchronization using dynamic state matching. The system 100 uses a Phase Locking Value (PLV) algorithm to synchronize the breath rates of the facilitator and user. PLV measures the phase alignment between two oscillatory signals, in this case, breath rates, and adjusts the facilitator's breath rate accordingly. A phase alignment score that is calculated by the system 100 for this purpose is defined as: $PLV \left(t\right) = \frac{1}{N} {\sum }_{K = 1}^{N} {e}^{j \left({ϕ}_{user}\left(tk\right)-{ϕ}_{facilitator}\left(tk\right)\right)}$ where:
▪ *ϕᵤₛₑᵣ*(*tk*) and *ϕ_{facilitαtor}*(*tk*) are the instantaneous phases of the breath rates for the user and facilitator, respectively.
▪ The PLV score ranges from 0 (no synchronization) to 1 (perfect synchronization).

Based on the calculations, a dynamic feedback is given to the facilitator to match the breathing pattern of the user. The facilitator's breath rate is visually represented as a sinusoidal wave overlaid with the participant's wave, along with dynamic feedback (e.g., "Breathe slower" or "Breathe deeper") to guide the facilitator. The system 100 provides a visual representation of each modality's alignment and the overall rapport score. This is displayed as dynamic graphs and alignment indicators on the screen. The system 100 may also use color-coded indicators, for example, green for high alignment, red for low alignment and so on, to provide immediate visual feedback to the facilitator.

The persistent emotional state for a pre-defined time interval is detected by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising: calculating a topological persistence score of the emotional state at each time instant by mapping the emotional states to a topological space, wherein the emotional state and a congruence score at a time instant is determined based on the computed weight for each multi-modal input data using a unified state model, wherein the congruence score is represented as: $CS \left(E\right) = 1 / N {\sum }_{i = 1}^{n} \left(1-\left\{\left|{X}_{i}-{B}_{i}\right|/σi\right\}\right) . {W}_{i} ,$ where, *Xᵢ* is the current value of the modality *i, Bᵢ* is the baseline of the modality *i, σi* is the standard deviation of *i*, and *Wᵢ* is the weight assigned to modality *i* based on its reliability. The cumulative alignment score is generated from the weight computed for the multi-modal input data of the user and the facilitator using a dynamic time warping and phase alignment technique.

Once the synchronization is achieved, then the system 100 generates the adaptive question for the facilitator to ask the user. In an embodiment, a plurality of adaptive questions are generated, which together form an adaptive questionnaire. Steps involved in the process of generating the adaptive questionnaire are explained below.

For this purpose, the system 100 uses a combination of Generative AI (alternately referred to as "Gen-AI") and multimodal data inputs (e.g., micro-expressions, breath rate, voice speed, magic words) to dynamically generate context-aware questions that elicit targeted emotional responses from the user. This system 100 takes into account the emotional and physiological state of the user in real-time, along with key linguistic patterns (e.g., magic words), to craft personalized questions that drive deeper engagement, build rapport, and uncover underlying emotions. The facilitator can use these questions to guide the interaction in a more meaningful and effective manner.

For dynamically generating the context-aware questions, the system 100 receives continuous input, including:
∘ Emotional State Shifts: State shift alerts and emotional intensity scores from the micro-expression and voice analysis feeds.
∘ Physiological Data: Breath rate and heart rate variability are analysed to determine the user's physiological state (e.g., calm, stressed).
∘ Magic Words: Specific key phrases and emotionally significant words identified by the Generative AI are stored and weighted based on their frequency and contextual relevance.

The system 100 performs a contextual analysis on the received inputs to synthesize inputs from various modalities, creating a comprehensive understanding of the user's current state. The context includes:
▪ Current Emotional State: Derived from micro expression and voice analysis.
▪ Physiological State: Derived from breath rate and other physiological inputs.
▪ Recent State Shifts: Analysis of recent state transitions (e.g., from calm to anxious) and their respective TPS values.
▪ Magic Word Context: Words that hold emotional weight for the user (e.g., "family," "success," "failure", and the like).

The system 100 maintains a dynamic state transition graph that tracks the user's emotional trajectory over time. This graph is used to predict possible future emotional states that would allow the Gen-AI to tailor questions accordingly.

From the contextual data identified by means of the contextual analysis, the system 100 uses a suitable Gen-AIGen-AI model such as ChatGPT, by augmenting the Gen-AI model with context and state data. The Gen-AI model is provided with a custom prompt that incorporates the current context, emotional state, and magic words. The prompt is dynamically constructed using:

User Emotion: {Current Emotional State} Recent Emotional Shifts: {Transition Details} Magic Words: {List of Magic Words} Facilitator State: {Facilitator's Emotional State}

The Gen-AI model uses this multimodal data to continuously evaluate the user's emotional state in real time. Based on the current state and recent state shifts, the Gen-AI model determines the most appropriate line of questioning:
∘ The Gen-AI model generates questions that align with the user's current state stability. If a stable but negative state (e.g., prolonged anxiety) is detected with a high Topological Persistence Score (TPS), the system 100 generates questions that gently probe the root causes without reinforcing the negative state, and once the user anchors it, gently shift the conversation towards a more positive or neutral topic.
∘ For transient or unstable states (low TPS), the Gen-AI model generates questions that shift the conversation to explore new topics or emotional triggers, thereby encouraging transitions to more stable, positive states. If the user is in a positive or resourceful emotional state, the system 100 generates questions that reinforce and deepen this state, using identified magic words and contextually relevant topics.

The system 100 employs state-based thresholds for each emotional and physiological indicator. These thresholds determine when the current line of questioning should be adjusted or when the conversation should transition to a new topic. The thresholds are dynamically adjusted based on real-time feedback from the user:
∘ Thresholds for Emotional Intensity: If the intensity of negative emotions (e.g., anger, sadness) exceeds a predefined threshold, the system 100 generates questions that redirect the user's focus away from triggering topics.
∘ Thresholds for Physiological Indicators: For example, if the breath rate remains elevated for a sustained period, indicating prolonged stress, the system 100 introduces calming questions or suggest a topic change.

The system 100 uses a Transition Logic Framework (TLF) to determine when to shift the conversation to a new topic. The logic is based on several factors:
∘ Emotional State Persistence: If a negative state persists despite attempts to shift it (e.g., repeated anxious expressions and increased heart rate), the system 100 recognizes this resistance and transitions to a less challenging topic.
∘ Contextual Relevance and Magic Words: When a topic resonates strongly with the user (indicated by frequent use of magic words), the system 100 explores the topic further until the emotional intensity decreases, signaling that the user has sufficiently engaged with or resolved the issue.

After each question, the system 100 monitors the user's reaction using a Feedback Analysis Loop. This loop evaluates changes in micro-expressions, voice tone, and physiological states to determine if the question had the desired effect. The system 100 uses this feedback to decide the next course of action.

The system 100 calculates a State Stability Score S(E) by integrating the Topological Persistence Score (TPS) with deviations from the baseline values of each modality. The value of S(E) indicates stability of a state over a period of time. This approach ensures that the state stability score reflects both topological stability and congruence with the user's physiological and emotional data. $S \left(E\right) = \frac{1}{T} {\sum }_{t = 1}^{T} \left\{TPS\left(E\right)∗{\sum }_{i = 1}^{n} {w}_{i} ⋅ \left(\left|{X}_{i}\left(t\right)-{B}_{i}\right|/\left\{{σ}_{i}+ε\right\}\right)\right\}$ where,
- T is the time window over which the stability score is calculated.
- *TPS*(*E*) is the Topological Persistence Score of emotional state E.
- *wᵢ* is the dynamic weight of modality i, adjusted based on the consistency and relevance of the input.
- *Xᵢ*(*t*) is the current value of modality *i* at time t.
- *Bᵢ* is the baseline value of modality i.
- *σᵢ* is the standard deviation of modality i.
- *ε* is a small constant to prevent division by zero.

The State Stability Score *S*(*E*) increases with higher TPS, indicating that the emotional state is well-supported by persistent topological features and consistent multimodal data. Each emotional state E has a State Stability Score *S*(*E*) calculated based on the duration and consistency of the emotional state across all modalities.

The system 100 also computes a State Transition Probability score using the TPS of a current state E and the expected impact of the generated question Q, as: $P \left(E\to \left|E′\right|\right) = 1 / \left(1+{e}^{- \left(impact\left(Q,E′\right)-S\left(E\right)\right)}\right)$ where,
∘ *impact*(*Q,E'*) is an expected impact of question *Q* on transitioning the user's emotional state from *E* to *E".*
∘ *S*(*E*) is the State Stability Score of the current emotional state *E.*

After each question is asked, the system 100 uses the multimodal inputs to monitor changes in the user's emotional state and updates the TPS accordingly. If the TPS of the target state E' increases (indicating that the question was effective), the system 100 continues along the same line of questioning or explores deeper aspects of the topic. If the TPS decreases (indicating resistance or a negative reaction), the system 100 shifts the topic or rephrases the question using different contextual cues. This feedback loop enables Gen-AI model to dynamically adapt its questioning strategy based on real-time emotional state changes and topological stability.

Further, the system 100 calculates the change in the user's TPS before and after each question to determine the effectiveness of the question in stabilizing or shifting the emotional state, as: $Δ TPS \left(E\right) = {TPS}_{post} \left(E-\right) - {TPS}_{pre} \left(E-\right)$

If ΔTPS(E) > 0, it indicates that the question contributed to the stabilization or positive shift of the state. Based on the feedback analysis, the Gen-AI model dynamically adjusts the subsequent questions:
∘ If ΔTPS(E) is positive: The Gen-AI model continues to explore the current topic, using deeper questions to further stabilize the positive state or encourage positive state transitions.
∘ If ΔTPS(E) is negative: The Gen-AI model generates questions that gently changes the line of questioning or reduce the intensity of the current state to prevent reinforcing negative emotions.
∘ Anchoring: The system 100 prompt the facilitator to anchor identified emotional states based on the TPS Score.

Further, at step 210 of the method 200, the system 100 displays, via the one or more hardware processors, a generative game for the user to play based on the detected persistent emotional state, wherein the user plays the generative game until the user is identified to be in a pre-defined state. The generative game is a personalized game based on the user's preferences, personality traits, and real-time physiological and emotional states. The game leverages principles from neurolinguistic programming (NLP) and require coordination of eye-hand movements, concentration, and cognitive engagement. The system 100 monitors the user's performance and dynamically adjusts game difficulty to help reach a pre-defined state, i.e., a high-performance state (e.g., flow state). Once the user is identified to be in the high-performance state, the system 100 uses dynamic anchoring to reinforce and anchor these states, making it easier for the user to re-access these peak performance levels in the future. Once the user is identified to be in the pre-defined state, i.e., in one of the high-performance states, the system 100 generates one or more adaptive questions based on the user response for the displayed adaptive game. In an embodiment, the user response for the displayed adaptive game indicates to the system 100 whether the user is in the pre-defined state, and in turn the system 100 generates the one or more adaptive questions.

The system 100 uses a Generative AI model (Gen-AI model), such as GPT-based, to create game scenarios and elements that are tailored to the user's personality, preferences, and current emotional state. The Gen-AI model leverages real-time data to determine the user's likes, dislikes, and personality traits. These inputs are used to design personalized games that incorporate elements such as colour schemes, difficulty levels, and types of challenges, words used in the game and other elements of the games. The system 100 continuously receives input from multiple modalities (e.g., micro-expressions, breath rate, voice analysis) to dynamically adjust game scenarios. For example, if a user is detected to be in a low energy state, the system 100 presents a game that involves more active engagement, such as eye-hand coordination or concentration tasks that involve visual and auditory stimuli or the pace of the game will be adjusted. The system 100 analyses linguistic patterns, magic words, and preferences captured during a gaming session during which the user was engaged in gameplay, and accordingly, behavioural profile is assigned to the user. Based on the behavioural profile, the system 100 selects or generates game types that align with the user's strengths and interests, thereby increasing engagement and motivation. The games include activities that require eye-hand coordination, such as:
∘ Eye Tracking Tasks: The system 100 tracks eye movements using API and generates tasks where the user has to follow moving objects or quickly react to visual cues.
∘ Hand Coordination: Tasks may involve synchronizing or following instructions on the screen. The system 100 monitors reaction time, accuracy, and focus level.
∘ Leg Coordination Tasks: Using a camera-based motion tracking system, the games can include tasks that require users to move their legs or change their posture in response to visual or auditory cues.

The system 100 also generates games that require sustained concentration and cognitive engagement with words, colors, math, objects and other elements.

During game play, the system 100 continuously monitors the user's performance using a combination of metrics, including:
∘ Reaction Time: Time taken to respond to visual or auditory stimuli.
∘ Accuracy: Precision in performing the tasks (e.g., hitting targets, following eye-tracking cues).
∘ Concentration Level: Derived from voice analysis, breath rate stability, eye ball tracking and micro-expression analysis to detect cognitive load or distractions.

The system 100 detects the High-Performance State using the Topological Persistence Score (TPS). In this process, the system 100 calculates a High-Performance State Score based on the Topological Persistence Score (TPS) of the user's physiological and emotional data during gameplay. The TPS score captures the stability and duration of features such as breath rate, heart rate variability, and micro-expression intensity, indicating whether the user is in a flow state or experiencing peak cognitive and physical performance. 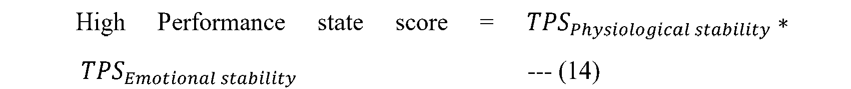 High Performance state score = TPSPhysiological stability * TPSEmotional stability --- (14)

The system 100 detects when the TPS of these states reaches a certain threshold, indicating that the user is in a high-performance state.

The system 100 also adjusts game difficulty in real-time. If the user is detected to be approaching a high-performance state, the system 100 dynamically increases game difficulty or introduces additional challenges to push the user to their peak capacity without inducing stress or cognitive overload. If the user shows signs of declining performance or cognitive fatigue (e.g., reduced TPS or slower reaction times), the system 100 decreases the difficulty to maintain engagement and prevent burnout. In an embodiment, the game difficulty may be varied in steps, i.e., easy, average, difficult, very difficult, etc.

Details on the dynamic anchoring mechanism that the system 100 uses to reinforce and anchor the states is explained herein. Once the system 100 identifies that the user has reached the high-performance state based on TPS and performance metrics, it initiates the dynamic anchoring mechanism to associate this state with specific sensory cues (e.g., visual or auditory stimuli). The system 100 dynamically selects anchoring cues based on the user's sensory preferences and current state, such as:
▪ Visual Cues: Bright colors, shapes, or dynamic visual patterns.
▪ Auditory Cues: A specific tone or background music that plays consistently whenever the user reaches a high-performance state.

When the high-performance state is detected, the system 100 plays the selected cue repeatedly, establishing a neural association between the high-performance state and the cue. As the user experiences the high-performance state multiple times during gameplay, the association is strengthened, making it easier for the user to access this state in the context where they felt not in high performance before. The system 100 provides real-time feedback on the anchoring process by displaying the state score and the associated cue on the screen, allowing the user to become consciously aware of their high-performance state. This feedback reinforces the anchoring process and enhances the effectiveness of the neural association.

Further, at step 212 of the method 200, the system 100 generates, via the one or more hardware processors 102, a customized interview structure for the user, based on the adaptive question generated prior to displaying the generative game, the detected persistent state, a user response for the displayed adaptive game, one or more adaptive questions generated based on the user response for the displayed adaptive game, and the anchor for one or more states identified to be shifted. The customized interview structure serves as an adaptive response that is displayed to the facilitator, who can then ask questions to the user.

Once the questions are asked to the user, the system 100 mirrors a pre-interview structure in the post-interview process. The pre-interview structure refers to the question(s) that are asked the user prior to making the user play the generative game, whereas the post-interview process refers to asking the one or more questions that are generated based on the one or more adaptive questions generated based on the user response for the displayed adaptive game. The system 100 uses the Gen-AI model (e.g., ChatGPT) to ask the same contextual questions in both the pre and post-interviews, and analyses multimodal inputs to validate changes in emotional and cognitive states. The TPS score is used to quantify state stability and intensity across both interviews, ensuring robust validation of state shifts and a reliable assessment of intervention effectiveness. The system 100 compares the emotional intensity using TPS scoring. The system 100 calculates difference in emotional intensity and stability for each state between the pre- and post-interview, based on change in TPS: $Δ TPS \left(E\right) = {TPS}_{post} \left(E\right) - {TPS}_{pre} \left(E\right)$

A positive ΔTPS(E) indicates that the emotional state has become more stable or persistent, while a negative value suggests a reduction in intensity or instability.

If the post-interview reveals a shift towards resourceful states, the gen-AI model generates future pacing questions. These questions ask the user to imagine future scenarios similar to the ones discussed in the pre-interview and visualize how they would respond differently given their newly stabilized emotional state. For example: "Now that you feel more confident managing deadlines, how do you see yourself approaching the next big project?". The system 100 performs a direct comparison of the TPS values and emotional intensity scores from the pre-interview and post-interview sessions. This comparison validates whether the user's emotional state has shifted significantly after the intervention. A validation score is calculated as: $Validation Score = Δ TPS \left(E\right) / {TPS}_{pre} \left(E\right) + ϵ$

A positive validation score indicates that the user's emotional state has improved (e.g., decreased anxiety or increased resourcefulness).

The system 100 integrates multimodal data such as micro-expressions, breath rate, and voice tonality to validate state shifts observed in the TPS comparison. If the multimodal data corroborates the TPS-based state shifts, the system 100 confirms the state transition and proceeds to future pacing. At this stage, a plurality of future pacing questions like user to imagine future scenarios similar to the adaptive question generated prior to displaying the generative game, i.e. in a pre-interview stage, are generated and asked to the user, and the user is made to visualize how the user would respond differently given their newly stabilized emotional state, i.e., what is achieved by the user after playing the generative game. If discrepancies are detected, additional questions are asked to explore the cause of the discrepancy.

The system 100 uses the Gen-AI model to generate a narrative summary of the user's state before and after the intervention, incorporating insights from the pre- and post-interview and changes in TPS. The report is aligned to a Psychological Wellbeing (PWB) Scale and that measures six aspects of wellbeing and happiness: autonomy, environmental mastery, personal growth, positive relations with others, purpose in life, and self-acceptance, and includes:
∘ Emotional State Changes: Summary of which emotional states improved or regressed.
∘ Intensity and Stability Shifts: Detailed analysis of changes in emotional intensity and state stability.
∘ Before and After on the PWB Scale of Autonomy, environmental mastery, purpose, relationships and other.

Some of the use-cases of the emotion detection are given below:

### Corporate Change Management:

Application: Organizational change, such as mergers or restructuring, can be a significant source of stress and anxiety for employees. A digital behavioral health solution can be integrated into corporate wellness programs to support employees during these transitions, providing tailored support to manage emotional challenges.

Benefits: The solution can help employees navigate organizational changes more effectively, promoting mental well-being and resilience, and ultimately enhancing employee engagement and retention.

### Recruitment, Training, and Onboarding:

Application: The digital mental behavioral health solution can be used in the recruitment, training and onboarding process to gain a deeper understanding of candidates' emotional and cognitive abilities. By analyzing facial expressions, physiological data, and performance on cognitive exercises, the solution can provide valuable insights into a candidate's problem-solving skills, emotional intelligence, and overall fit for a particular role or organizational culture.

Benefits: This information can be used to make more informed hiring decisions and support the onboarding process, ensuring a smooth transition for new hires and promoting their long-term success within the organization

### Chronic Disease Management:

Application: Individuals with chronic physical conditions often face significant mental health challenges. The digital behavioral health solution can provide real time personalized support by integrating facial expression analysis, physiological monitoring, and AI-powered interventions to address the unique mental health needs of these individuals. It is preventive solution which can help the patients be mentally ready to face the health issues and keep a track of their mental health. This solution can be implemented in hospitals and it can be made as a process of their routine check.

Benefits: A user (patient in this context) being in a peaceful mental state has a crucial positive impact in treatment of chronic diseases. This can be achieved by leveraging multimodal biomarkers and AI-driven interventions. The method of the present disclosure can help chronic disease patients manage their mental health without relying on traditional medicines or interventions, thereby improving their overall well-being and quality of life.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of emotional state shift detection of a user. The embodiment, thus provides a mechanism for video and audio based emotional state shift detection. Moreover, the embodiments herein further provide a mechanism for customizing questions to be asked to a user, based on the detected emotion state of the user.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:
capturing (202), via one or more hardware processors, a multi-modal input data in real time, at each time stamp over a period of time, from a user and a facilitator during an interactive session using a set of sensors;
pre-processing (204), via the one or more hardware processors, the multi-modal input data to generate a pre-processed input data, wherein the pre-processing comprises of filtering noise from the multi-modal input data using a set of filtering techniques;
computing (206), via the one or more hardware processors, a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics;
generating (208), via the one or more hardware processors, an adaptive question for the facilitator based on a cumulative alignment score indicating rapport between the user and the facilitator, a persistent emotional state detected based on the computed weight of each multi-modal input data, and a predicted future emotional state;
displaying (210), via the one or more hardware processors, a generative game for the user to play based on the detected persistent emotional state, wherein the user plays the generative game until the user is identified to be in a pre-defined state; and
generating (212), via the one or more hardware processors, a customized interview structure for the user, based on the adaptive question generated prior to displaying the generative game, the detected persistent state, a user response for the displayed adaptive game, one or more adaptive questions generated based on the user response for the displayed adaptive game, and an anchor for one or more states identified to be shifted.

2. The processor implemented method as claimed in claim 1, wherein the multi-modal input data comprises micro-expressions, gestures, heart rate, breather rate, breathe volume, eye ball tracking and eye gesture data, and one or more additional parameters representing emotional state of the user.

3. The processor implemented method as claimed in claim 1, wherein the sensitivity factor is represented as: ${S}_{i} \left(t\right) = \left\{\left({α}_{i}∗\left‖{X}_{i}\left(t\right)-{B}_{i}\left(t\right)\right‖\right)/{σ}_{i}{\left(t\right)}^{2}+ϵ\right\} + \left\{{β}_{i}/\left(\begin{matrix}1 + \\ Noiselevel \left({X}_{i}\left(t\right)\right)\end{matrix}\right)\right\} ,$ where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data *i* over a defined time window, which measures input stability, *βᵢ* is a coefficient that controls the contribution of noise levels, *Noiselevel*(*Xᵢ*(*t*)) is the detected noise level for the multi-modal input data *i* at time t, and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$

4. The processor implemented method as claimed in claim 1, wherein the persistent emotional state for a pre-defined time interval is detected by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising:
calculating a topological persistence score of the emotional state at each time instant by mapping the emotional states to a topological space,
wherein the emotional state and a congruence score at a time instant is determined based on the computed weight for each multi-modal input data using a unified state model, wherein the congruence score is represented as: $CS \left(E\right) = 1 / N {\sum }_{i = 1}^{n} \left(1-\left\{\left|{X}_{i}-{B}_{i}\right|/\left.σi\right)\right\}\right) . {W}_{i} ,$ where *Xᵢ* is the current value of the modality *i, Bᵢ* is the baseline of the modality *i,* σᵢ is the standard deviation of *i,* and *Wᵢ* is the weight assigned to modality *i* based on its reliability.

5. The processor implemented method as claimed in claim 1, wherein the cumulative alignment score is generated from the weight computed for the multi-modal input data of the user and the facilitator using a dynamic time warping and phase alignment technique.

6. The processor implemented method as claimed in claim 1, wherein the future emotional state is predicted at each time instant from the weight computed for the multi-modal input data of the user, the adaptive question generated at the previous time instant, a user response generated at the previous time instant, and an anchor generated at the previous instant.

7. A system (100), comprising:
one or more hardware processors (102);
a communication interface (112); and
a memory (104) storing a plurality of instructions, wherein the plurality of instructions cause the one or more hardware processors to:
capture a multi-modal input data in real time, at each time stamp over a period of time, from a user and a facilitator during an interactive session using a set of sensors;
pre-process the multi-modal input data to generate a pre-processed input data, wherein the pre-processing comprises of filtering noise in the multi-modal input data using a set of filtering techniques;
compute a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics;
generate an adaptive question for the facilitator based on a cumulative alignment score indicating rapport between the user and the facilitator, a persistent emotional state detected based on the computed weight of each multi-modal input data, and a predicted future emotional state;
display a generative game for the user to play based on the detected persistent emotional state, wherein the user plays the generative game until the user is identified to be in a pre-defined state; and
generate a customized interview structure for the user, based on the adaptive question generated prior to displaying the generative game, the detected persistent state, a user response for the displayed adaptive game, one or more adaptive questions generated based on the user response for the displayed adaptive game, and the anchor for one or more states identified to be shifted.

8. The system as claimed in claim 7, wherein the multi-modal input data comprises micro-expressions, gestures, heart rate, breather rate, breathe volume, eye ball tracking and eye gesture data, and one or more additional parameters representing emotional state of the user.

9. The system as claimed in claim 7, wherein the sensitivity factor is represented as: 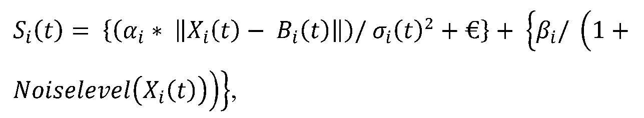 where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data *i* over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$

10. The system as claimed in claim 7, wherein the one or more hardware processors are configured to detect the persistent emotional state for a pre-defined time interval by performing a topological data analysis using (i) a plurality of emotional states determined at each time instant of the pre-defined time interval, (ii) an adaptive question generated at a previous time instant and a state predicted for the user after the generated adaptive question, (iii) a user response generated for the adaptive question at the previous time instant, (iv) an anchor generated at the previous instant, and (v) context of conversation, comprising:
calculating a topological persistence score of the emotional state at each time instant by mapping the emotional states to a topological space,
wherein the emotional state and a congruence score at a time instant is determined based on the computed weight for each multi-modal input data using a unified state model, wherein the congruence score is represented as: $CS \left(E\right) = 1 / N {\sum }_{i = 1}^{n} \left(1-\left\{\left|{X}_{i}-{B}_{i}\right|/\left.σi\right)\right\}\right) . {W}_{i} ,$ where Xᵢ is the current value of the modality i, Bᵢ is the baseline of the modality i, σᵢ is the standard deviation of i, and Wᵢ is the weight assigned to modality i based on its reliability.

11. The system as claimed in claim 7, wherein the one or more hardware processors are configured to generate the cumulative alignment score from the weight computed for the multi-modal input data of the user and the facilitator using a dynamic time warping and phase alignment technique.

12. The system as claimed in claim 7, wherein the one or more hardware processors are configured to predict the future emotional state at each time instant from the weight computed for the multi-modal input data of the user, the adaptive question generated at the previous time instant, a user response generated at the previous time instant, and an anchor generated at the previous instant.

13. One or more non-transitory machine-readable information storage mediums comprising one or more instructions which when executed by one or more hardware processors cause:
capturing a multi-modal input data in real time, at each time stamp over a period of time, from a user and a facilitator during an interactive session using a set of sensors;
pre-processing the multi-modal input data to generate a pre-processed input data, wherein the pre-processing comprises of filtering noise from the multi-modal input data using a set of filtering techniques;
computing a weight of each multi-modal input data of the user and the facilitator based on a sensitivity factor, wherein the sensitivity factor is determined by performing dynamic sensitivity adjustment for each multi-modal input data based on a baseline level, a noise level, a contextual relevance of the multi-modal input data, a plurality of environmental parameter changes, and a plurality of individual user characteristics;
generating an adaptive question for the facilitator based on a cumulative alignment score indicating rapport between the user and the facilitator, a persistent emotional state detected based on the computed weight of each multi-modal input data, and a predicted future emotional state;
displaying a generative game for the user to play based on the detected persistent emotional state, wherein the user plays the generative game until the user is identified to be in a pre-defined state; and
generating a customized interview structure for the user, based on the adaptive question generated prior to displaying the generative game, the detected persistent state, a user response for the displayed adaptive game, one or more adaptive questions generated based on the user response for the displayed adaptive game, and an anchor for one or more states identified to be shifted.

14. The one or more non-transitory machine readable information storage mediums as claimed in claim 13, wherein the multi-modal input data comprises micro-expressions, gestures, heart rate, breather rate, breathe volume, eye ball tracking and eye gesture data, and one or more additional parameters representing emotional state of the user.

15. The one or more non-transitory machine readable information storage mediums as claimed in claim 13, wherein the sensitivity factor is represented as: 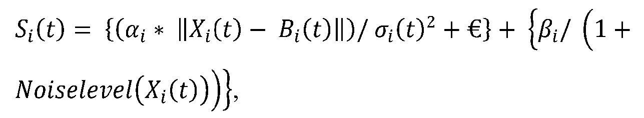 where, *Sᵢ*(*t*) is the sensitivity factor of each multi-modal input data, *αᵢ* is a modality-specific scaling coefficient that adjusts the weight based on the multi-modal input data importance, ∥*Xᵢ*(*t*) - *Bᵢ*(*t*)∥ represents deviation of a current input *Xᵢ*(*t*) from a personalized baseline *Bᵢ*(*t*) which indicates how different the current state is from the expected norm, *σᵢ*(*t*)²is the variance of the multi-modal input data *i* over a defined time window, which measures input stability, *βᵢ* is a coefficient that controls the contribution of noise levels, *Noiselevel*(*Xᵢ*(*t*)) is the detected noise level for the multi-modal input data *i* at time *t,* and wherein the weight for each multi-modal input data is computed using, ${w}_{j} \left(t\right) = \left\{{S}_{i}\left(t\right)\right\} / \left\{{\sum }_{j = 1}^{n} {S}_{j} \left(t\right)\right\} .$
